# EUROPÄISCHE PATENTANMELDUNG

(11) **EP 0 935 962 A1**
(43) Veröffentlichungstag der Anmeldung: **18.08.1999**
(21) Anmeldenummer: 99101700.5
(22) Anmeldetag: 09.02.1999
(51) Int. Cl.: A61K 31/385

(54) **Verwendung von 5-(1,2-Dithiolan-3-yl-)valeriansäure (.alpha.-Liponsäure) oder deren physiologisch verträgliche Salze zur Behandlung von Störungen des Fettstoffwechsels**

(30) Priorität: 10.02.1998 DE 19806354
(71) Anmelder: BiRD Berolina innovative Research and Development GmbH, 16341 Zepernick (DE); esparma GmbH, 39045 Magdeburg (DE)
(72) Erfinder: Alken, Rudolf-Giesbert, Dr., 16341 Zepernick (DE); Koegst, Dieter, 39175 Wahlitz (DE); Fries, Gerhard, Dr., 39175 Wahlitz (DE)
(74) Vertreter: Müller, Thomas

(57) **Zusammenfassung**

Es wird die Verwendung von 5-(1,2-Dithiolan-3-yl-)valeriansäure (α-Liponsäure) oder deren physiologisch verträgliche Salze zur Herstellung von Arzneimitteln zur Behandlung von Störungen des Fettstoffwechsels beschrieben.

## Beschreibung

Die Erfindung betrifft die Verwendung von 5-(1,2-Dithiolan-3-yl-)valeriansäure (α-Liponsäure) oder deren physiologisch verträgliche Salze zur Herstellung von Arzneimitteln zur Behandlung von Störungen des Fettstoffwechsels.

Arteriosklerose ist nach Herzkrankheiten und Krebserkrankungen eine der Haupttodesursachen bei Menschen. Ein Risikofaktor für die Erkrankung an Arteriosklerose ist eine erhöhte Menge an Serumlipiden und Lipoproteinen, so daß eine Senkung der Serumlipid- und Lipoproteinspiegel für die Therapie wichtig ist.

Aufgabe der vorliegenden Erfindung ist es daher, einen Wirkstoff zur Verfügung zu stellen, welcher zur Behandlung von Störungen des Fettstoffwechsels geeignet ist.

Die Aufgabe wird erfindungsgemäß durch die Verwendung von 5-(1,2-Dithiolan-3-yl-)valeriansäure (α-Liponsäure) oder deren physiologisch verträgliche Salze zur Herstellung von Arzneimitteln zur Behandlung von Störungen des Fettstoffwechsels.

Erfindungsgemäß bevorzugt ist die Verwendung zur Behandlung von Hyperlipidämie und/oder Hyperlipoproteinämie` insbesondere von Hypercholesterinämie und/oder Hypertriglyceridämie.

Überraschenderweise wurde gefunden, daß α-Liponsäure zur Behandlung von Störungen des Fettstoffwechsels wirksam ist.

Sowohl bei normalen Blutfettwerten, als auch bei Dyslipoproteinämien führt die Verwendung von α-Liponsäure zu einer signifikanten Verringerung der Blutfettwerte. Dieser Befund ist auch davon unabhängig, ob eine gleichzeitige Hypertonie medikamentös behandelt wird.

Die α-Liponsäure wird zur Behandlung der Polyneuropathie, vorzugsweise der diabetischen Polyneuropathie in Dosen um 600 mg/Tag eingesetzt. Die Verträglichkeit der oralen und parenteralen Therapie in diesen Dosen ist sehr gut. Die wirksamen Dosen zur Behandlung von Fettstoffwechselstörungen liegen in der gleichen Größenordnung und sind deshalb gleich gut verträglich.

α-Liponsäure ist erstmals von Reed et al. (Science 114, S. 93 (1951)) isoliert worden und wird seit 1961 als Arzneimittel bei diabetischer Polyneuropathie und Polyneuritis verwendet.

Die Herstellung der α-Liponsäure ist an sich bekannt und beispielsweise beschrieben in US-PS 2,980,716 und US-PS 3,049,549

Neben der freien α-Liponsäure können erfindungsgemäß auch deren physiologisch verträgliche Salze zur erfindungsgemäßen Behandlung verwendet werden.

Geeignete Salze lassen sich in an sich bekannter Weise aus der α-Liponsäure unter Zusatz von Basen herstellen. Geeignete Basen sind beispielsweise Metallhydroxide, insbesondere Alkali- und Erdalkalihydroxide und organische Säuren, wie beispielsweise Ethylendiamin, Trometamol und Methylglucamin.

Die Salze werden in der Regel in an sich bekannter Weise durch Mischen der freien Base oder deren Lösungen mit der α-Liponsäure oder deren Lösungen in einem organischen Lösungsmittel, beispielsweise einem niederen Alkohol wie Methanol, Ethanol, n-Propanol oder Isopropanol oder einem niederen Keton wie Aceton, Methylethylketon oder Methyl-isobutylketon oder einem Ether wie Diethylether, Tetrahydrofuran oder Dioxan, erhalten. Zur besseren Kristallabscheidung können auch Mischungen der genannten Lösungsmittel verwendet werden. Die Salze der α-Liponsäure können in an sich bekannter Weise auch mit Ionenaustauschern hergestellt werden.

Gegenstand der vorliegenden Erfindung sind auch Arzneimittel zur oralen, rektalen, subcutanen, intravenösen oder intramuskulären Applikation, die neben üblichen Träger- und Verdünnungsmitteln α-Liponsäure oder deren physiologisch verträglichen Salzen als Wirkstoff enthalten.

Die Arzneimittel der Erfindung werden mit den üblichen festen oder flüssigen Trägerstoffen oder Verdünnungsmitteln und den üblicherweise verwendeten pharmazeutischtechnischen Hilfsstoffen entsprechend der gewünschten Applikationsart mit einer geeigneten Dosierung in bekannter Weise hergestellt. Die bevorzugten Zubereitungen bestehen in einer Darreichungsform, die zur oralen Applikation geeignet ist. Solche Darreichungsformen sind beispielsweise Tabletten, Filmtabletten, Dragees, Kapseln, Pillen, Pulver, Lösungen oder Suspensionen oder Depotformen.

Selbstverständlich kommen auch parenterale Zubereitungen wie Injektionslösungen in Betracht. Weiterhin seien als Zubereitungen beispielsweise auch Suppositorien genannt.

Entsprechende Tabletten können beispielsweise durch Mischen des Wirkstoffs mit bekannten Hilfsstoffen, beispielsweise inerten Verdünnungsmitteln wie Dextrose, Zucker, Sorbit, Mannit, Polyvinylpyrrolidon, Sprengmitteln wie Maisstärke oder Alginsäure, Bindemitteln wie Stärke oder Gelantine, Gleitmitteln wie Magnesiumstearat oder Talk und/oder Mitteln zur Erzielung eines Depoteffektes wie Carboxylpolymethylen, Carboxylmethylcellulose, Celluloseacetatphthalat oder Polyvinylacetat, erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Polyvinylpyrrolidon oder Schellack, Gummiarabicum, Talk, Titandioxid oder Zucker, hergestellt werden. Dabei kann auch die Drageehülle aus mehreren Schichten bestehen, wobei die oben bei den Tabletten erwähnten Hilfsstoffe verwendet werden können.

Lösungen oder Suspensionen mit dem erfindungsgemäßen Wirkstoff können zusätzlich geschmacksverbessernde Mittel wie Saccharin, Cyclamat oder Zucker sowie z. B. Aromastoffe wie Vanillin oder Orangenextrakt enthalten. Sie können außerdem Suspendierhilfsstoffe wie Natriumcarboxymethylcellulose oder Konservierungsstoffe wie p-Hydroxy-benzoate enthalten. Wirkstoffe enthaltende Kapseln können beispielsweise hergestellt werden, indem man den Wirkstoff mit einem inerten Träger wie Milchzucker oder Sorbit mischt und in Gelatinekapseln einkapselt.

Geeignete Suppositorien lassen sich beispielsweise durch Vermischen mit dafür vorgesehenen Trägermitteln wie Neutralfetten oder Polyäthylenglykol bzw. deren Derivaten herstellen.

Das folgende Beispiel erläutert die Erfindung:

### Beispiel

Im Rahmen einer offenen, nicht intervenierenden Anwendungsbeobachtungsstudie wurden 1729 Patienten mit Polyneuropathien und gleichzeitiger Hypertonie untersucht, die überwiegend eine Kombinationstherapie von α-Liponsäure und Captopril erhielten (n=1289). Eine etwas kleinere Gruppe wurde mit α-Liponsäure, nicht mit Captopril therapiert (n=280). Der Behandlungszeitraum erstreckte sich über ca. 3 Monate. α-Liponsäure wurde überwiegend in der Dosierung von 600 mg pro Tag oral oder parenteral gegeben, Captopril wurde oral in Tagesdosen von 12.5 bis 50 mg eingenommen.

Zu Beginn der Therapie und zum Ende der Beobachtung nach 3 Monaten wurden fakultativ die Blutfettwerte notiert. In der Auswertung der Gesamtpopulation zeigte sich eine statistisch auffällige Senkung der Triglyceride und des Gesamt- und LDL-Cholesterols sowie ein leichter Anstieg des HDL-Cholesterols.

Aufgrund dieser Ergebnisse wurden aus dieser Anwendungsbeobachtung die Patienten, die pathologische Laborwerte des Lipidstoffwechsels aufwiesen, untersucht. Wegen der unterschiedlichen Basisbehandlung wurden hierzu die Patienten mit einer Kombinationstherapie α-Liponsäure und Captopril sowie einer Monotherapie α-Liponsäure subgruppiert: Die 160 verbleibenden Patienten (mit Monotherapie Captopril, n=154; ohne Angaben zur Therapie, n=6) wurden nicht in die weitere Betrachtung einbezogen, da in dieser Restgruppe nur eine geringe, nicht vergleichbare Anzahl von Patienten Ausgangswerte entsprechend den unter Punkt 2 genannten Bereichen aufwies (Triglyceride n=0, Gesamtcholesterol n=63, HDL-Cholesterol n=0, LDL-Cholesterol n=32).

In einer ersten Betrachtung wurden die Parameter unabhängig voneinander dargestellt, d.h. pro Parameter wurden alle Patienten einbezogen, die in diesem Parameter die unten darge-stellten Ausgangswerte aufwiesen:
- Triglyceride: > 6 mmol/l;
- Gesamtcholesterol: > 6 mmol/l;
- HDL-Cholesterol: 0.7 - 0.8 mmol/l;
- LDL-Cholesterol: > 2.5 mmol/l.

Die Grenzen der Bereiche wurden in Anlehnung an in der Literatur berichtete Studien gewählt (H. Thuro, Effektive Cholesterin- und Triglyceridsenkung mit Atorvastatin, Beilage in Der Internist 1996; 37 (8), 1-8).

Die Datenlage bei den Triglyceriden stellt sich wie folgt dar:

| | | | | |
|---|---|---|---|---|
| Gesamtpatientenzahl mit α-Liponsäure-Therapie: | | | | 1569 |
| Anzahl Patienten mit Vor- oder Endwert: | | | | 1094 |
| mit Vorwert: | 1068, | davon: | ≥ 6 mmol/l | 37 |
| | | | < 6 mmol/l | 1031 |
| mit Endwert: | 671, | davon: | ≥ 6 mmol/I | 18 |
| | | | < 6 mmol/l | 653 |
| mit paarigen Daten: | 645. | | | |

Dabei zeigte sich eine Abnahme der Triglyceride in beiden Therapiegruppen. Über alle Patienten betrachtet, reduzierte sich der Mittelwert von 8,96 mmol/l (n=33) auf 7,16 mmol/l (n=26). Die Verringerung der Triglyceride über alle Patienten war signifikant bei 26 paarigen Beobachtungen (p<0,05). Der mittlere prozentuale Abfall der Triglyceride lag bei über 20% in allen Gruppen.

Die Datenlage beim Gesamtcholesterol war wie folgt:

| | | | | |
|---|---|---|---|---|
| Gesamtpatientenzahl mit α-Liponsäure-Therapie: | | | | 1569 |
| Anzahl Patienten mit Vor- oder Endwert: | | | | 1239 |
| mit Vorwert: | 1210, | davon: | ≥ 6 mmol/l | 853 |
| | | | < 6 mmol/l | 357 |
| mit Endwert: | 793, | davon: | ≥ 6 mmol/I | 490 |
| | | | < 6 mmol/l | 303 |
| mit paarigen Daten: | 764. | | | |

Bei insgesamt 853 Patienten mit erhöhten Gesamtcholesterol-Werten vor Therapie (Mittelwert 7,32 mmol/l) sanken die Werte auf 6,57 mmol/l, wobei bei insgesamt 570 Patienten paarige Werte vorlagen. Bei diesen war die Bewegung signifikant (p<0,0001). Bei einer Besetzung von 500 Patienten mit paarigen Werten in der Gruppe mit kombinationstherapie und 70 Patienten in der Gruppe mit Mono-therapie zeigte die Abnahme des Gesamtcholesterols ebenfalls hohe Signifikanz (p<0,0001). Die mittlere prozentuale Abnahme lag bei ca. 10% in allen Gruppen.

Die Datenlage beim HDL-Cholesterol war wie folgt:

| | | | | |
|---|---|---|---|---|
| Gesamtpatientenzahl mit α-Liponsäure-Therapie: | | | | 1569 |
| Anzahl Patienten mit Vor-oder Endwert: | | | | 576 |
| mit Vorwert: | 540, | davon: | > 8 mmol/l | 28 |
| | | | ≤ 8 mmol/l | 512 |
| mit Endwert: | 360, | davon: | > 8 mmol/I | 12 |
| | | | ≤ mmol/l | 348 |
| mit paarigen Daten: | 324. | | | |

Beim HDL-Cholesterol zeigte sich in beiden Gruppen ein geringer Anstieg der Werte. Über alle Patienten bewegte sich das HDL-Cholesterol im Mittel von 0,76 mmol/l (n=33) auf 0,97 mmol/l (n=13). Die Zunahme zeigte in der Gesamtsowie in den Untergruppen keine Signifikanz (Kombinationstherapie n=9, Monotherapie n=4). Die prozentuale Zunahme des HDL-Cholesterols lag im Mittel bei über 20%.

Die Datenlage beim LDL-Cholesterol war wie folgt:

| | | | | |
|---|---|---|---|---|
| Gesamtpatientenzahl mit α-Liponsäure-Therapie: | | | | 1569 |
| Anzahl Patienten mit Vor-oder Endwert: | | | | 391 |
| mit Vorwert: | 369, | davon: | ≥ 2,5 mmol/l | 336 |
| | | | < 2,5 mmol/l | 33 |
| mit Endwert: | 251, | davon: | ≥ 2,5 mmol/I | 226 |
| | | | < 2,5 mmol/l | 25 |
| mit paarigen Daten: | 229. | | | |

Beim LDL-Cholesterol zeigte sich eine Abnahme der Werte in beiden Therapiegruppen. Über alle Patienten betrachtet, reduzierte sich der Mittelwert von 4,41 mmol/l (n=336) auf 4,03 mmol/l (n=208). Die Verringerung des LDL-Cholesterols über alle Patienten war signifikant bei 208 paarigen Beobachtungen (p<0,001). Die in den Subgruppen beobachtete Abnahme zeigte bei einem Vorliegen von 183 paarigen Werten in der Kombinationsgruppe und 25 paarigen Werten in der Monogruppe ebenfalls Signifikanz (Kombination p<0,0001, Mono p<0,001). Die prozentuale Abnahme des LDL-Cholesterols lag im Mittel bei ca. 10% in allen Gruppen.

Bei allen Parametern war die beobachtete Veränderung der Werte unter Therapie in der Gruppe der Patienten mit einer Monotherapie von α-Liponsäure stärker ausgeprägt im Vergleich mit der Gruppe der Patienten, die eine Kombinationstherapie mit Captopril erhielten.

In einer weiteren Betrachtung wurden die Patienten entsprechend den 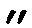 Richtlinien der Deutschen Gesellschaft zur Bekämpfung von Fettstoffwechselstörungen und ihrer Folgeerkrankungen DGFF (Lipid-Liga) e.V." (Beilage zum Deutschen Ärzteblatt 1996; 4, 1-8) unabhängig von der Therapieform anhand ihres Krankheitsbildes drei Gruppen zugeordnet:
1) isolierte LDL-Hypercholesterinämie
   (LDL > 4,14, Triglyceride ≤ 2,2 mmol/l);
2) kombinierte LDL-Hypercholesterinämie
   (LDL > 4,14, Triglyceride > 2.2 mmol/I);
3) isolierte Hypertriglyceridämie
   (LDL ≤ 4,14, Triglyceride > 2,2 mmol/l).

Hierbei wurden als Grenzwerte Normwerte aus der Literatur gesetzt (Wilson et al., Harrisons's principles of international medicine 12^{th} edition, McGraw-Hill, 1991).

Die erste Gruppe war mit 62, die zweite Gruppe mit 112 und die dritte Gruppe mit 83 Patienten belegt.

Die Ausgangs-Datenlage bei den Triglyceriden war wie folgt:

| | | | | |
|---|---|---|---|---|
| Gesamtpatientenzahl mit α-Liponsäure-Therapie: | | | | 1569 |
| Anzahl Patienten mit Vor-oder Endwert: | | | | 1094 |
| mit Vorwert: | 1068, | davon: | > 2,2 mmol/l | 560 |
| | | | ≤ mmol/l | 508 |
| mit Endwert: | 671, | davon: | > 2,2 mmol/I | 270 |
| | | | ≤ mmol/l | 401 |
| mit paarigen Daten: | 645. | | | |

Die Absolutwerte der Triglyceride lagen in der ersten Gruppe im Mittel bei 1,69 mmol/l vor Beginn der Therapie und stiegen im Therapieverlauf geringfügig auf 1,70 mmol/l (n=38). Die Zunahme war nicht signifikant. Die zweite Gruppe lag in ihren Absolutwerten etwas über denen der dritten Gruppe mit isolierter Hypertriglyceridämie und zeigte einen stärkeren Abfall (Vorwert 3,75 mmol/l, Endwert 2,74 mmol/l. Bei einem Vorliegen von 91 paarigen Werten war diese Differenz sowohl absolut (0.83 mmol/l) als auch prozentual (20%) bei einem p<0,0001 statistisch hochsignifikant. In der dritten Gruppe gingen die Werte von 3,33 mmol/l (n=83) um ca. 16% auf 2,69 mmol/l zurück (p<0,0001, n=67).

Die Ausgangs-Datenlage beim Gesamtcholesterol war wie folgt:

| | | | | |
|---|---|---|---|---|
| Gesamtpatientenzahl mit α-Liponsäure-Therapie: | | | | 1569 |
| Anzahl Patienten mit Vor-oder Endwert: | | | | 1239 |
| mit Vorwert: | 1210, | davon: | > 6,2 mmol/l | 764 |
| | | | ≤ mmol/l | 446 |
| mit Endwert: | 793, | davon: | > 6,2 mmol/l | 405 |
| | | | ≤ mmol/l | 388 |
| mit paarigen Daten: | 764. | | | |

Die Absolutwerte beim Gesamtcholesterol lagen in der ersten Gruppe mit einem mittleren Ausgangswert von 7,01 mmol/l (n=62) und einem Endwert von 6,49 mmol/l (n=40) geringfügig unter denen der Einzelparameterdarstellung mit 7,32 und 6,57 mmol/l, die Abnahme betrug 8% gegenüber 10%. In der Gruppe zwei lagen Vor- und Endwert (7,71 und 6,86 mmol/l, n=112 und 94) etwas über denen der Einzelwertdarstellung, wobei die Abnahme hier mit 10% etwa gleich war. Die Gruppe drei wies deutlich geringere Werte auf. Der Ausgangswert war jedoch mit 6,31 mmol/l (n=83) noch pathologisch. Er sank unter Therapie um 5% auf 5,88 mmol/l (n=67) in den grenzwertigen Bereich.

Die Ausgangs-Datenlage beim HDL-Cholesterol war wie folgt:

| | | | | |
|---|---|---|---|---|
| Gesamtpatientenzahl mit α-Liponsäure-Therapie: | | | | 1569 |
| Anzahl Patienten mit | | Vor- oder Endwert: | | 576 |
| mit Vorwert: | 540, | davon: | < 0,9 mmol/l | 66 |
| | | | ≥ mmol/l | 474 |
| mit Endwert: | 360, | davon: | < 0,9 mmol/I | 34 |
| | | | ≥ mmol/l | 326 |
| mit paarigen Daten: | 324. | | | |

Die Absolutwerte beim HDL-Cholesterol lagen in allen drei Gruppen (Gruppe 1: Vorwert 1,29 mmol/1 (n=61), Endwert 1,34 mmol/l (n=34); Gruppe 2: Vorwert 1,15 mmol/l (n=108), Endwert 1,15 mmol/l (n=77); Gruppe 3: 1,19 mmol/l (n=83), Endwert 1,31 mmol/l (n=59)) deutlich über denen der Einzelparameterdarstellung. Dementsprechend lagen die absoluten und prozentualen Zunahmen ebenfalls deutlich niedriger, wobei die Zunahme in der Gruppe zwei mit 4,5% nur sehr geringfügig war. Gruppe eins zeigte eine Zunahme um ca. 10%, die mit 34 paarigen Werten keine Signifikanz zeigte. Am stärksten war die Erhöhung des HDL-Cholesterols mit ca. 15% in Gruppe drei ausgeprägt, hier ließ sich bei einem Vorliegen von 59 paarigen Werten statistisch Signifikanz nachweisen (p<0,01).

Die Ausgangs-Datenlage beim LDL-Cholesterol war wie folgt:

| | | | | |
|---|---|---|---|---|
| Gesamtpatientenzahl mit α-Liponsäure-Therapie: | | | | 1569 |
| Anzahl Patienten mit Vor- oder Endwert: | | | | 391 |
| mit Vorwert: | 369, | davon: | > 4,14 mmol/l | 179 |
| | | | ≤ mmol/l | 190 |
| mit Endwert: | 251, | davon: | > 4,14 mmol/I | 91 |
| | | | ≤ mmol/l | 160 |
| mit paarigen Daten: | 229. | | | |

Aufgrund des erhöhten Grenzwertes lagen die Werte beim LDL-Cholesterol in den Gruppen eins (Vorwert 5,02 mmol/l; Endwert 4,33 mmol/l, n=33) und zwei (Vorwert 5,42 mmol/l; Endwert 4,75 mmol/l, n=74) höher als in den obigen Darstellungen (Ausgangswert 4,41 mmol/l, Endwert 4,03 mmol/l). In beiden Gruppen war die mittlere absolute und prozentuale Abnahme (16%, 14%) des LDL-Cholesterols mit einem p<0,0001 hochsignifikant. Der mittlere Ausgangswert der Gruppe drei befand sich mit 3,29 mmol/l innerhalb des Normbereiches. Der Endwert dieser Gruppe lag mit 3,23 mmol/l (n=59) ebenfalls unter dem Ausgangswert, obwohl in dieser Gruppe keine LDL-Hypercholesterinämie vorlag. Die Veränderung ergab jedoch keine Signifikanz.

Wie zu erwarten war, lag das Verhältnis von LDL- zu HDL-Cholesterol in den Gruppen mit einer bestehenden LDL-Hypercholesterinämie (Gruppe eins und zwei) im kritischen Bereich, wobei der Quotient in der Gruppe der Patienten mit gemischter Hyperlipidämie sowohl vor als auch nach Therapie deutlich über dem der Gruppe mit isolierter LDL-Hypercholesterinämie lag (isoliert: Vorwert 3,89, Endwert 3,23; gemischt: Vorwert 4,71, Endwert 4,13). In beiden Gruppen zeigte sich ein deutlicher Abfall des Verhältnisses von LDL- zu HDL-Cholesterol im Verlauf der Therapie (isoliert um 16,97%, gemischt um 12,31%), es blieb jedoch innerhalb des kritischen Bereiches. In der Gruppe der Patienten ohne LDL-Cholesterinämie lag der Ausgangswert im Normbereich, verbesserte sich jedoch ebenfalls um 10,51%.

Ein Vergleich der Belegung der Klassifikationsgruppen bei Patienten mit initialem Dyslipoproteinämiebefund vor und nach Therapie zeigt eine Abnahme der Gruppe mit kombinierter Hypercholesterinämie zugunsten der isolierten Formen.

## Patentansprüche

1. Verwendung von 5-(1,2-Dithiolan-3-yl-)valeriansäure (α-Liponsäure) oder deren physiologisch verträgliche Salze zur Herstellung von Arzneimitteln zur Behandlung von Störungen des Fettstoffwechsels.

2. Verwendung gemäß Anspruch 1 zur Behandlung von Hyperlipidämie und/oder Hyperlipoproteinämie.

3. Verwendung gemäß Anspruch 2 zur Behandlung von Hypercholesterinämie und/oder Hypertriglyceridämie.

4. Pharmazeutische Zusammensetzung enthaltend 5-(1,2-Dithiolan-3-yl-)valeriansäure (α-Liponsäure) oder deren physiologisch verträgliche Salze zur Behandlung von Störungen des Fettstoffwechsels neben pharmazeutisch verträglichen Hilfs- und/oder Zusatzstoffen.
